# EUROPÄISCHE PATENTSCHRIFT

(11) **EP 2 234 962 B1**
(45) Veröffentlichungstag und Bekanntmachung des Hinweises auf die Patenterteilung: **16.10.2013**
(21) Anmeldenummer: 08864439.8
(22) Anmeldetag: 10.12.2008
(51) Int. Cl.: C07C 213/00, C07C 215/08

(54) **VERFAHREN ZUR HERSTELLUNG VON (S)-2-AMINO-1-PROPANOL (L-ALANINOL) AUS (S)-1-METHOXY-2-PROPYLAMIN**
METHOD FOR PRODUCING (S)-2-AMINO-1-PROPANOL (L-ALANINOL) FROM (S)-1-METHOXY-2-PROPYLAMINE
PROCÉDÉ DE FABRICATION DE (S)-2-AMINO-1-PROPANOL (L-ALANINOL) À PARTIR DE (S)-1-MÉTHOXY-2-PROPYLAMINE

(30) Priorität: 20.12.2007 EP 07150226
(43) Veröffentlichungstag der Anmeldung: 06.10.2010
(73) Patentinhaber: BASF SE, 67056 Ludwigshafen (DE)
(72) Erfinder: KUTZKI, Olaf, 68167 Mannheim (DE); DITRICH, Klaus, 67161 Gönnheim (DE); BARTSCH, Michael, CH-8816 Hirzel (CH)
(86) Internationale Anmeldenummer: PCT/EP2008/067181
(87) Internationale Veröffentlichungsnummer: WO 2009/080505

(56) Entgegenhaltungen:
- WO-A-2005/077871
- KURIHARA, YOSHIE ET AL: "Addition reaction of dinitrogen trioxide with cinnamyl acetate" BULLETIN OF THE CHEMICAL SOCIETY OF JAPAN, 38(8), 1327 -30 CODEN: BCSJA8; ISSN: 0009-2673, 1965, XP002515489 in der Anmeldung erwähnt
- SCHREYER, R. C.: "Synthesis of 3-amino-1,2-propanediol and 2,3-diamino-1-propanol" JOURNAL OF THE AMERICAN CHEMICAL SOCIETY, 73, 4404 -5 CODEN: JACSAT; ISSN: 0002-7863, 1951, XP008102394 in der Anmeldung erwähnt

## Beschreibung

Die Erfindung betrifft ein Verfahren zur Herstellung von (S)-2-Amino-1-propanol (L-Alaninol) aus (S)-1-Methoxy-2-propylamin über das Hydrochlorid des (S)-2-Amino-1-propanols und anschließender Aufarbeitung.

Es besteht ein großer Bedarf an (S)-2-Amino-1-propanol, das als Zwischenprodukt für pharmazeutische Wirkstoffe wie z.B. dem Antibiotikum Levofloxacin von großer Bedeutung ist.

(S)-2-Amino-1-propanol ist bereits bekannt und kann nach unterschiedlichen Verfahren hergestellt werden. Ausgangsstoff für die ersten Verfahren zur Herstellung von (S)-2-Amino-1-propanol sind Derivate der Aminosäure L-Alanin gewesen. So wird von Karrer et al. in Helv. Chim. Acta 1948, 31, 1617 und in JP-A 6199747 die Reduktion von den entsprechenden Estern der Aminosäure L-Alanin zum (S)-2-Amino-1-propanol beschrieben.

Weitere Reduktionen wurden ausgehend von den Thioestern des L-Alanins von Jeger et al. in Helv. Chim. Acta 1946 29, 684 beschrieben. Die Reduktion des entsprechenden L-Alanins ist von M.K.Ghorai et al. in Tetrahedron Lett. 2007, 48, 2471 sowie in WO 2005/077871 beschrieben. Auch die Reduktion von Amidderivaten des L-Alanins sind von I. Schön et al. in J. Org. Chem. 1983, 48, 1916 bereits offenbart. M. Studer et al. beschreiben in Adv. Synth. Catal. 2001, 343, 802 die katalytische Hydrierung eines Alaninesters zum (S)-2-Amino-1-propanol.

Als weitere Möglichkeit zur Herstellung von (S)-2-Amino-1-propanol wird in WO-A2 99/07199 und in WO-A2 01/73038 die enzymatische Überführung von 2-Aminopropan in (S)-2-Amino-1-propanol beschrieben.

Die Spaltung von Methylethern durch die Verwendung von Salzsäure ist sowohl bei Normaldruck von Schreyer et al. in J. Am. Chem. Soc. 1951, 73, 4404 beschrieben als auch unter erhöhtem Druck von Kurihara et al. in Bull. Chem. Soc. Jpn. 1965, 38, 1327.

Nachteilig an all diesen beschriebenen Verfahren sind zum einen der Einsatz von Metallhydriden für die Reduktionen der einzelnen Aminosäurederivate, da diese sehr teuer sind und auch schwer aus dem anschließend entstandenem Produkt zu entfernen und zum anderen bei den enzymatischen Verfahren die sehr langen Reaktionszeiten, die geringen Ausbeuten sowie die großen Verdünnungen, in denen gearbeitet werden muss. Nachteilig an der katalytischen Hydrierung von Ester-Derivaten des Alanins ist die große Menge an benötigtem Katalysator.

Die Aufgabe der vorliegenden Erfindung ist daher ein gegenüber dem bisherigen Stand der Technik möglichst kostengünstiges Verfahren bereit zustellen, das es ermöglicht (S)-2-Amino-1-propanol trotzdem mit ähnlich hohen ee-Werten und in ähnlich guten Ausbeuten wie im Stand der Technik beschrieben zu erhalten.

Diese Aufgabe wird gelöst durch ein Verfahren zur Herstellung von (S)-2-Amino-1-propanol umfassend folgende Schritte:
I) Umsetzung von (S)-1-Methoxy-2-propylamin mit mindestens 2 Äquivalenten einer 30 bis 40 Gew.-%igen Salzsäure, entweder
   la) bei Temperaturen von größer 80°C in einem Autoklaven bei Drücken im Bereich von 3 bis 45 bar für 1 bis 12 Stunden und anschließender Abkühlung auf Raumtemperatur und Entspannung des Autoklaven
      oder
   Ib) es wird für 30 bis 60 Stunden bei Normaldruck zum Rückfluss erhitzt,
II) anschließend wird das wässrige Lösungsmittel aus Schritt Ia) oder aus Schritt Ib) abdestilliert
III) danach wird das Reaktionsprodukt aus Schritt II) entweder
   IIIa) mit einer anorganischen Base versetzt bis ein pH-Wert größer 10 eingestellt worden ist
      oder
   IIIb) das aus Schritt II erhaltene Produkt wird mit einem Gemisch aus einem höher siedenden Lösungsmittel und einer stärkeren Base umgesetzt,
IV) anschließend wird
   entweder
   IVa) das Reaktionsprodukt aus Schritt IIIa vom Wasser destillativ befreit und der Rückstand wird mit einem Lösungsmittel versetzt und anschließend filtriert,
      oder
   IVb) das Reaktionsprodukt aus Schritt IIIa wird mit einem Gemisch aus einem Azeotrop bildenden organischen Lösungsmittel und einem höher siedenden Verdünnungsmittel versetzt und Wasser und (S)-2-Aminopropan-1-ol werden zusammen mit dem Azeotrop bildenden organischen Lösungsmittel azeotrop abdestilliert, anschließend werden die (S)-2-Aminopropan-1-ol enthaltenden Destillationsfraktionen vereinigt.
   V) das aus Schritt IVa) erhaltene Filtrat oder die aus Schritt IVb) erhaltenen vereinigten Filtrate oder die aus Schritt IIIb) erhaltenen Mischungen werden destilliert.

Vorteilhaft ist das erfindungsgemäße Verfahren, wenn eine 35 bis 38 %ige Salzsäure in Schritt I eingesetzt wird.

Vorteilhaft ist das erfindungsgemäße Verfahren, wenn bei einer Temperatur im Bereich von 80 bis 100 °C die Umsetzung in Schritt Ia) mehr als 9 Stunden dauert und der Druck zwischen 3 bis 5 bar liegt.

Vorteilhaft ist das erfindungsgemäße Verfahren, wenn bei einer Temperatur im Bereich von mehr als 100 °C die Umsetzung in Schritt Ia) weniger als 9 Stunden dauert und der Druck zwischen 15 bis 45 bar liegt.

Vorteilhaft ist das erfindungsgemäße Verfahren, wenn bei einer Temperatur im Bereich von 130 bis 135 °C die Umsetzung in Schritt Ia) weniger als 4,5 Stunden dauert und der Druck zwischen 19-30 bar liegt.

Vorteilhaft ist das erfindungsgemäße Verfahren, wenn gemäß Schritt Ib) für 45-60 Stunden zum Rückfluss erhitzt wird.

Vorteilhaft ist das erfindungsgemäße Verfahren, wenn gemäß Schritt Ib) nach 15 Stunden weitere 1-2 Äquivalente einer 30 bis 40 Gew.-%igen Salzsäure zugegeben werden und insgesamt für 30-50 Stunden zum Rückfluss erhitzt wird.

Vorteilhaft ist das erfindungsgemäße Verfahren, wenn die in Schritt IIIa) eingesetzte anorganische Base ausgewählt ist aus der Gruppe von Natriumhydroxid, Kaliumhydroxid, Lithiumhydroxid, Calciumhydroxid, Natriumcarbonat, Kaliumcarbonat, Kaliumhydrogencarbonat oder Natriumhydrogencarbonat.

Vorteilhaft ist das erfindungsgemäße Verfahren, wenn der pH-Wert in Schritt IIIa größer gleich 12 ist.

Vorteilhaft ist das erfindungsgemäße Verfahren, wenn das in Schritt IIIb eingesetzte höher siedende Lösungsmittel ausgewählt ist aus der Gruppe von Glykolen mit 2 bis 8 Kohlenstoffatomen wie Ethylenglykol, Diethylenglykol und Triethylenglykol oder Polyalkylenglykolen, wie z. B. Produkten der Marken Pluriol E und P^{®} (Alkoxilate aus Ethylenoxid oder Propylenoxid der Fa. BASF AG), insbesondere Pluriol^{®} P600 (Polypropylenoxid der Fa. BASF AG, kinematische Viskosität ca. 130 mm²/s bei 20°C), oder Produkten der Marke Lutron^{®} (modifizierte Polyglykolether der Fa. BASF AG), insbesondere Lutron^{®} HF1 (modifizierter Polyglykolether der Fa. BASF AG, kinematische Viskosität 220-280 mm²/s bei 23°C), oder Aminoalkoholen wie Ethanolamin, Diethanolamin und Triethanolamin oder Gemischen dieser Lösungsmittel sowie deren Gemische mit Wasser.

Vorteilhaft ist das erfindungsgemäße Verfahren, wenn die in Schritt IIIb eingesetzte stärkere Base ausgewählt ist aus der Gruppe von Alkalihydroxiden wie Natriumhydroxid, Kaliumhydroxid oder Erdalkalihydroxiden wie Calciumhydroxid oder Alkalialkoholaten wie Natriummethanolat, Natriumethanolat, Kaliumethanolat, Natrium-tert.-butanolat und Kalium-tert.-butanolat oder Diazabicyclooctan (DABCO), Diazabicyclononan (DBN), Diazabicycloundecan (DBU) und Tri-n-octylamin.

Vorteilhaft ist das erfindungsgemäße Verfahren, wenn das Lösungsmittel, das in Schritt IVa) eingesetzt wird, ausgewählt ist aus der Gruppe von Methanol, Ethanol, n-Propanol, iso-Propanol, n-Butanol, 2-Butanol (sec- Butanol) 2-Methyl-1-propanol (iso-Butanol), Ethylacetat, Methylacetat oder Dichlormethan. Ganz besonders bevorzugt ist Methanol.

Vorteilhaft ist das erfindungsgemäße Verfahren, wenn das Azeotrop bildende organische Lösungsmittel in Schritt IVb) ausgewählt ist aus der Gruppe von Toluol, o-, mund p-Xylol sowie Ethylbenzol und deren Gemischen, bevorzugt sind o-, m- und p-Xylol und deren technische Gemische, die bis zu 25 Gew.-% Ethylbenzol enthalten können.

Vorteilhaft ist das erfindungsgemäße Verfahren, wenn die Destillation in Schritt V bei einem Druck von 2 bis 6 mbar durchgeführt wird.

Für das erfindungsgemäße Verfahren wird (S)-1-Methoxy-2-propylamin mit Salzsäure zum (S)-2-Amino-1-propanol Hydrochlorid umgesetzt und anschließend wird durch entsprechende Aufarbeitung das (S)-2-Amino-1-propanol freigesetzt gemäß folgender Reaktion:

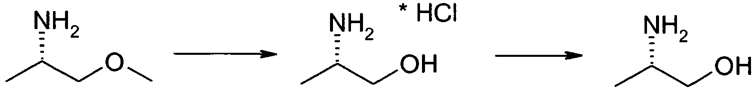

Für die Bildung des (S)-2-Amino-1-propanol Hydrochlorids wird (S)-1-Methoxy-2-propylamin mit mindestens 2 Äquivalenten einer 30 bis 40 Gew.-%igen Salzsäurelösung, besonders bevorzugt einer 35 bis 38 Gew.-%igen Salzsäurelösung, ganz besonders bevorzugt einer 37Gew.-%igen Salzsäurelösung umgesetzt. Bevorzugt ist die Zugabe von 2 bis 5 Äquivalenten der 30 bis 40 Gew.-%igen Salzsäure.

Die Umsetzung mit der Salzsäure kann auf zwei unterschiedliche Weisen erfolgen, die aber beide zum Hydrochlorid des (S)-2-Amino-1-propanol führen.

In der einen Variante wird nach der vollständigen Zugabe der Salzsäure das erhaltene Gemisch in einen Autoklaven gegeben. Anschließend wird bei Temperaturen von größer 80°C, bevorzugt ≥ 90°C ganz besonders bevorzugt im Bereich von 135 bis 140°C und bei Drücken von 3 bis 45 bar, bevorzugt 19 bis 30 bar für 1 bis 12 Stunden, bevorzugt ≤ 10 Stunden, ganz bevorzugt ≤ 4 Stunden gerührt. Nach der erfolgten Reaktion im Autoklaven wird der Autoklav auf Raumtemperatur abgekühlt und anschließend entspannt.

In der anderen Reaktionsdurchführung wird nach der Zugabe des (S)-1-Methoxy-2-propylamins in die Salzsäure das erhaltene Gemisch anschließend für 30 bis 60 Stunden, bevorzugt sind 45 bis 50 Stunden zum Rückfluss erhitzt, wobei in Zeitintervallen von 10-20 Stunden jeweils weitere 0 -2 Äquivalente einer 30 bis 40 Gew.-%igen Salzsäure zugegeben werden.

Zur Herstellung des (S)-2-Amino-1-propanol Hydrochlorids ist die Umsetzung im Autoklaven gegenüber der Erhitzung unter Rückfluss bevorzugt.

Das nach der Umsetzung im Autoklaven oder das nach Erhitzung unter Rückfluss erhaltene Produkt wird anschließend durch Destillation vom Wasser befreit.

Das so hergestellte Hydrochlorid des (S)-2-Amino-1-propanol wird anschließend bei Temperaturen von weniger als 60 °C, bevorzugt im Bereich von 10 bis 40 °C gekühlt. Zu dieser gekühlten Lösung wird anschließend eine wässrige Lösung einer anorganischen Base unter Rühren zugetropft, bis die Lösung einen pH-Wert größer 10, bevorzugt größer 12, ganz besonders bevorzugt größer 14 aufweist. Die anorganischen Basen sind hierbei ausgewählt aus der Gruppe von Natriumhydroxid, Kaliumhydroxid, Lithiumhydroxid, Calciumhydroxid, Natriumcarbonat, Kaliumcarbonat, Kaliumhydrogencarbonat oder Natriumhydrogencarbonat. Besonders bevorzugt verwendet man Natriumhydroxid oder Kaliumhydroxid. Die anorganische Base wird vorzugsweise als wässrige Lösung in Konzentrationen zwischen 10 und 50 Gew.-% eingesetzt.

Die so hergestellte Lösung kann nun unterschiedlich aufgearbeitet werden. In einer Variante wird das Wasser unter vermindertem Druck abdestilliert. Hierbei bleibt ein Rückstand übrig, der in einem Lösungsmittel aufgenommen wird. Das Lösungsmittel ist dabei ausgewählt aus der Gruppe von Methanol, Ethanol, n-Propanol, iso-Propanol, n-Butanol, 2-Butanol (sec- Butanol) 2-Methyl-1-propanol (iso-Butanol), Dichlormethan, Methylacetat, Ethylacetat. Anschließend wird diese Lösung filtriert.

Bei einer anderen Variante der Aufarbeitung wird die wässrige alkalische Lösung aus Schritt IIIa mit einem Gemisch aus einem Azeotrop bildenden organischen Lösungsmittel und einem höher siedenden Verdünnungsmittel versetzt. Das Azeotrop bildende organischen Lösungsmittel ist dabei ausgewählt aus der Gruppe von Toluol, o-, m- und p-Xylol sowie Ethylbenzol und deren Gemischen, bevorzugt sind o-, m- und p-Xylol und deren technische Gemische, die bis zu 25 Gew.-% Ethylbenzol enthalten können.

Als höher siedendes Verdünnungsmittel sind Verdünnungsmittel mit einem Siedepunkt größer 190 °C (bei 1013 mbar) sowie Gemische der entsprechenden Verdünnungsmittel sowie deren Gemische mit Wasser zu verstehen. Das höher siedende Verdünnungsmittel ist dabei ausgewählt aus der Gruppe von Polyalkylenglykolen, wie z. B. Produkten der Marken Pluriol E und P^{®} (Alkoxilate aus Ethylenoxid oder Propylenoxid der Fa. BASF AG), insbesondere Pluriol^{®} P600 (Polypropylenoxid der Fa. BASF AG, kinematische Viskosität ca. 130 mm²/s bei 20°C), oder Produkten der Marke Lutrone^{®} (modifizierte Polyglykolether der Fa. BASF AG), insbesondere Lutron^{®} HF1 (modifizierter Polyglykolether der Fa. BASF AG, kinematische Viskosität 220-280 mm²/s bei 23°C), oder aus der Gruppe von Silikonölen, wie z. B. Produkten der Marke Baysilonee^{®} M (Polydimethylsiloxane der Fa. Momentive Performance Materials), insbesondere Baysilone^{®} M200 (Polydimethylsiloxane der Fa. Momentive Performance Materials, kinematische Viskosität 200 mm²/s bei 20°C), oder Paraffinölen. Das Mischungsverhältnis von Azeotrop bildendem organischen Lösungsmittel zu höher siedendem Verdünnungsmittel liegt bevorzugt im Bereich von 1 zu 1 bis 1 zu 6, besonders bevorzugt im Bereich von 1 zu 1.

Anschließend wird aus diesem Gemisch zusammen mit dem Azeotrop bildenden organischen Lösungsmittel Wasser, (S)-2-Amino-1-propanol und ein azeotropes Gemisch aus diesen beiden Verbindungen abdestilliert. Die (S)-2-Amino-1-propanol enthaltenden Fraktionen werden anschließend wieder vereint.

Anschließend wird entweder das Filtrat des Reaktionsschrittes IVa) oder die (S)-2-Amino-1-propanol enthaltenden Fraktionen aus Schritt IVb) destilliert.

Als alternative Aufarbeitung kann auch das aus Schritt II erhaltene Produkt mit einem Gemisch aus einem höher siedenden Lösungsmittel und einer stärkeren Base umgesetzt werden, wobei anschließend das freie (S)-2-Amino-1-propanol mittels Destillation entfernt wird. Als höher siedendes Lösungsmittel sind Lösungsmittel mit einem Siedepunkt größer 190 °C (bei 1013 mbar) einsetzbar. Bevorzugte höher siedende Lösungsmittel sind Glykole mit 2 bis 8 Kohlenstoffatome wie Ethylenglykol, Diethylenglykol und Triethylenglykol oder Polyalkylenglykole, wie z. B. Produkten der Marken Pluriol E und P^{®} (Alkoxilate aus Ethylenoxid oder Propylenoxid der Fa. BASF AG), insbesondere Pluriol^{®} P600 (Polypropylenoxid der Fa. BASF AG, kinematische Viskosität ca. 130 mm²/s bei 20°C), oder Produkten der Marke Lutron^{®} (modifizierte Polyglykolether der Fa. BASF AG), insbesondere Lutron^{®} HF1 (modifizierter Polyglykolether der Fa. BASF AG, kinematische Viskosität 220-280 mm²/s bei 23°C), oder Aminoalkohole wie Ethanolamin, Diethanolamin und Triethanolamin oder Gemische dieser Lösungsmittel sowie deren Gemische mit Wasser.

Als stärkere Basen sind solche zu verstehen, die ausgewählt sind aus der Gruppe von Alkalihydroxiden wie Natriumhydroxid, Kaliumhydroxid oder Erdalkalihydroxiden wie Calciumhydroxid oder Alkalialkoholaten wie Natriummethanolat, Natriumethanolat, Kaliumethanolat, Natrium-tert.-butanolat und Kalium-tert.-butanolat oder Diazabicyclooctan (DABCO), Diazabicyclononan (DBN), Diazabicycloundecan (DBU) und Tri-n-octylamin.

Das nach dem erfindungsgemäßen Verfahren hergestellte (S)-2-Amino-1-propanol weist einen ee-Wert auf, der dem ee-Wert des eingesetzten (S)-1-Methoxy-2-propylamin entspricht und ist in Ausbeuten von > 50 % zu erhalten.

### Beispiele

### Reaktionsdurchführung 1 (erfindungsgemäß):

(S)-1-Methoxy-2-propylamin (53,5 g, 0,6 mol, ee > 99%) wird in 148 g (1,5 mol) 37 Gew.-%ige aq. Salzsäure gegeben, wobei die Zugabe so langsam erfolgt, dass die Temperatur unter 30°C bleibt. Das Reaktionsgemisch wird anschließend bei einer Temperatur von 135°C in einem mit Stickstoff inertisierten Autoklaven bei 19-30 bar Eigendruck vier Stunden gerührt. Der Ansatz wird nach der Reaktion auf Raumtemperatur abgekühlt und vorsichtig entspannt. Nach Abdestillieren des Wassers erhält man eine sehr viskose ölartige Flüssigkeit an (S)-2-Aminopropan-1-ol hydrochlorid (Vollumsatz It. NMR und GC).

### Reaktionsdurchführung 2 (erfindungsgemäß):

(S)-1-Methoxy-2-propylamin (53,5 g, 0,6 mol, ee > 99%) wird in 148 g (1,5 mol) 37 Gew.-%ige aq. Salzsäure gegeben, wobei die Zugabe so langsam erfolgt, dass die Temperatur unter 30°C gehalten wird. Das Reaktionsgemisch wird anschließend bei einer Temperatur von 90°C in einem mit Stickstoff inertisierten Autoklaven bei maximal 5 bar (der Druck wird immer bei Erreichen von 5 bar auf 3 bar entspannt) 10 Stunden gerührt. Der Ansatz wird nach der Reaktion auf Raumtemperatur abgekühlt und vorsichtig entspannt. Nach Abdestillieren des Wassers erhält man eine sehr viskose ölartige Flüssigkeit an (S)-2-Aminopropan-1-ol hydrochlorid (Vollumsatz lt. NMR und GC).

### Reaktionsdurchführung 3 (erfindungsgemäß):

(S)-1-Methoxy-2-propylamin (53,5 g, 0,6 mol, ee > 99%) wird langsam in 148 g (1,5 mol) 37 Gew.-%ige aq. Salzsäure gegeben, wobei die Temperatur unter 30°C gehalten wird. Das Reaktionsgemisch wird anschließend unter Rückfluss (Temp.: 100°C) für 48 Stunden gekocht. Der Ansatz wird dann auf Raumtemperatur abgekühlt. Nach Abdestillieren des Wassers erhält man eine sehr viskose ölartige Flüssigkeit an (S)-2-Aminopropan-1-ol hydrochlorid (Vollumsatz It. NMR und GC).

### Reaktionsdurchführung 4 (erfindungsgemäß):

(S)-1-Methoxy-2-propylamin (53,5 g, 0,6 mol, ee > 99%) wird langsam in 148 g (1,5 mol) 37 Gew.-%ige aq. Salzsäure gegeben, wobei die Temperatur unter 30°C gehalten wird. Das Reaktionsgemisch wird anschließend unter Rückfluss (Temp.: 100°C) für 15 Stunden gekocht bevor mit weiteren 70 g (0,71 mol) 37 Gew.-%iger aq. Salzsäure versetzt und unter Rückfluss (Temp.: 100°C) für weitere 20 Stunden gekocht wird. Der Ansatz wird dann auf Raumtemperatur abgekühlt. Nach Abdestillieren des Wassers erhält man eine sehr viskose ölartige Flüssigkeit an (S)-2-Aminopropan-1-ol hydrochlorid (Vollumsatz It. GC).
Analytik ((S)-2-Aminopropan-1-ol Hydrochlorid):
¹H-NMR (MeOD, 500 MHz): δ = 1.35 (d, 3H), 3.41 (m, 1 H), 3.60 (m, 1 H), 3.77 (m, 1 H).
¹³C-NMR (MeOD, 125 MHz): δ = 15 (s), 51 (m), 64 (s).

### Versuchsbeispiele:

| Nr | Reaktions-zeit [h] | Temperatur [°C] | Druck [bar] | Umsatz [GC-FI.-%] | Ausbeute [GC-FI.%] | Reaktionsdurchführung |
|---|---|---|---|---|---|---|
| 1 | 2 | 135 | 19 | 100 | 88,1 | 1 |
| 2 | 2 | 135 | 23 | 100 | 77,7 | 1 |
| 3 | 2 | 135 | 42 | 91,6 | 91,6 | 1 |
| 4 | 2 | 135 | 30 | 100 | 100 | 1 |
| 5 | 2 | 135 | 28 | 99,5 | 99,5 | 1 |
| 6 | 4 | 135 | 28 | 100 | 99,3 | 1 |
| 7 | 4 | 90 | 18 | 99,1 | 98,3 | 1 |
| 8 | 10 | 90 | 5 | 100 | 100 | 2 |
| 9 | 48 | 100 | 1 | 100 | 90 | 3 |
| 10 | 35 | 100 | 1 | 100 | 90 | 4 |

Freisetzen des freien Amins aus dem Hydrochlorid:

### Aufarbeitung 1(erfindungsgemäß):

(S)-2-Aminopropan-1-ol hydrochlorid (aus Reaktionsdurchführung 1, 2, 3 oder 4) wird mit 100 mL Wasser versetzt (pH= 0,76). Unter Rühren und Kühlen wird mit ca. 30ml 50Gew.-%iger aq. NaOH-Lösung der pH-Wert auf ca. 12 eingestellt. Der nach Abdestillieren (5 mbar) des Wassers verbleibende breiartige Rest (NaCl fällt aus) wird mit 100 mL Methanol versetzt und filtriert. Das Filtrat wird dann destillativ vom Methanol befreit, wodurch man 26,4 g (0,35 mol) (S)-2-Aminopropan-1-ol (mittels NMR+GC bestätigt) (ee > 99%) erhält.

### Aufarbeitung 2 (erfindungsgemäß):

(S)-2-Aminopropan-1-ol hydrochlorid (aus Reaktionsdurchführung 1, 2, 3 oder 4) wird mit 50 mL Wasser versetzt. Unter Rühren und Kühlen wird mit ca. 25ml 50Gew.-%iger aq. NaOH-Lösung der pH-Wert auf ca. 14 eingestellt. Nach der Zugabe von 50 g Lutron^{®} HF1 (modifizierter Polyglykolether der Fa. BASF AG, kinematische Viskosität 220-280 mm²/s bei 23°C) und 50 ml Xylol wird das (S)-2-Aminopropan-1-ol im Gemisch mit Wasser/Xylol abdestilliert. Die Produkt enthaltenen Fraktionen werden vereinigt und nach destillativer Entfernung (5 mbar) des Wassers/Xylols erhält man 22,89 g (0,30 mol) (S)-2-Aminopropan-1-ol (mittels NMR+GC bestätigt) (ee > 99%).

### Aufarbeitung 3 (erfindungsgemäß):

(S)-2-Aminopropan-1-ol hydrochlorid (aus Reaktionsdurchführung 1, 2, 3 oder 4) wird mit 50 g Pluriol^{®} P600 (Polypropylenoxid der Fa. BASF AG, kinematische Viskosität ca. 130 mm²/s bei 20°C) versetzt. Unter Rühren wird mit 0,6 mol (1 Äq.) Natriummethylat (30 Gew.-% in Methanol) versetzt und destilliert (2 mbar). Auf diese Weise erhält man 24,04 g (0,32 mol) (S)-2-Aminopropan-1-ol (mittels GC bestätigt).
Analytik (freie Base):
¹H-NMR (MeOD, 500 MHz): δ = 1.02 (d, 3H), 2.90 (m, 1 H), 3.26 (m, 1 H), 3.45 (m, 1 H).
¹³C-NMR (MeOD, 125 MHz): δ=19 (s), 49 (m), 69 (s).

## Patentansprüche

1. Verfahren zur Herstellung von (S)-2-Amino-1-propanol umfassend folgende Schritte:
I) Umsetzung von (S)-1-Methoxy-2-propylamin mit mindestens 2 Äquivalenten einer 30 bis 40 Gew.-%igen Salzsäure,
entweder
la) bei Temperaturen von größer 80°C in einem Autoklaven bei Drücken im Bereich von 3 bis 45 bar für 1 bis 12 Stunden und anschließender Abkühlung auf Raumtemperatur und Entspannung des Autoklaven
oder
Ib) es wird für 30 bis 60 Stunden bei Normaldruck zum Rückfluss erhitzt,
II) anschließend wird das wässrige Lösungsmittel aus Schritt Ia) oder aus Schritt Ib) abdestilliert
III) danach wird das Reaktionsprodukt aus Schritt II) entweder
IIIa) mit einer anorganischen Base versetzt bis ein pH-Wert größer 10 eingestellt worden ist
oder
IIIb) das aus Schritt II erhaltene Produkt wird mit einem Gemisch aus einem höher siedenden Lösungsmittel und einer stärkeren Base umgesetzt,
IV) anschließend wird
entweder
IVa) das Reaktionsprodukt aus Schritt IIIa vom Wasser destillativ befreit und der Rückstand wird mit einem Lösungsmittel versetzt und anschließend filtriert,
oder
IVb) das Reaktionsprodukt aus Schritt IIIa wird mit einem Gemisch aus einem Azeotrop bildenden organischen Lösungsmittel und einem höher siedenden Verdünnungsmittel versetzt und Wasser und (S)-2-Aminopropan-1-ol werden zusammen mit dem Azeotrop bildenden organischen Lösungsmittel azeotrop abdestilliert, anschließend werden die (S)-2-Aminopropan-1-ol enthaltenden Destillationsfraktionen vereinigt.
V) das aus Schritt IVa) erhaltene Filtrat oder die aus Schritt IVb) erhaltenen vereinigten Filtrate oder die aus Schritt IIIb) erhaltenen Mischungen werden destilliert.

2. Verfahren nach Anspruch 1, wobei eine 35 bis 38 %ige Salzsäure in Schritt I eingesetzt wird.

3. Verfahren nach einem der Ansprüche 1 bis 2, wobei bei einer Temperatur im Bereich von 80 bis 100 °C die Umsetzung in Schritt Ia) mehr als 9 Stunden dauert und der Druck zwischen 3 bis 5 bar liegt.

4. Verfahren nach einem der Ansprüche 1 bis 2, wobei bei einer Temperatur im Bereich von mehr als 100 °C die Umsetzung in Schritt Ia) weniger als 9 Stunden dauert und der Druck zwischen 15 bis 45 bar liegt.

5. Verfahren nach einem der Ansprüche 1 bis 2, wobei bei einer Temperatur im Bereich von 130 bis 135 °C die Umsetzung in Schritt Ia) weniger als 4,5 Stunden dauert und der Druck zwischen 19-30 bar liegt.

6. Verfahren nach einem der Ansprüche 1 bis 2, wobei gemäß Schritt Ib) für 45-60 Stunden zum Rückfluss erhitzt wird.

7. Verfahren nach einem der Ansprüche 1 bis 2, wobei gemäß Schritt Ib) nach 15 Stunden weitere 1-2 Äquivalente einer 30 bis 40 Gew.-%igen Salzsäure zugegeben werden und insgesamt für 30-50 Stunden zum Rückfluss erhitzt wird.

8. Verfahren nach einem der Ansprüche 1 bis 7, wobei die in Schritt IIIa) eingesetzte anorganische Base ausgewählt ist aus der Gruppe von Natriumhydroxid, Kaliumhydroxid, Lithiumhydroxid, Calciumhydroxid, Natriumcarbonat, Kaliumcarbonat, Kaliumhydrogencarbonat oder Natriumhydrogencarbonat.

9. Verfahren nach einem der Ansprüche 1 bis 8, wobei der pH-Wert in Schritt IIIa größer gleich 12 ist.

10. Verfahren nach einem der Ansprüche 1 bis 7, wobei das in Schritt IIIb eingesetzte höher siedende Lösungsmittel ausgewählt ist aus der Gruppe von Glykolen mit 2 bis 8 Kohlenstoffatomen wie Ethylenglykol, Diethylenglykol und Triethylenglykol oder Polyalkylenglykolen oder Aminoalkoholen wie Ethanolamin, Diethanolamin und Triethanolamin oder Gemischen dieser Lösungsmittel sowie deren Gemische mit Wasser.

11. Verfahren nach einem der Ansprüche 1 bis 7 und 10, wobei die in Schritt IIIb eingesetzte stärkere Base ausgewählt ist aus der Gruppe von Alkalihydroxiden wie Natriumhydroxid, Kaliumhydroxid oder Erdalkalihydroxiden wie Calciumhydroxid oder Alkalialkoholaten wie Natriummethanolat, Natriumethanolat, Kaliumethanolat, Natrium-tert.-butanolat und Kalium-tert.-butanolat oder Diazabicyclooctan (DABCO), Diaza-bicyclononan (DBN), Diazabicycloundecan (DBU) und Tri-n-octylamin.

12. Verfahren nach einem der Ansprüche 1 bis 9, wobei das Lösungsmittel, das in Schritt IVa) eingesetzt wird ausgewählt ist aus der Gruppe von Methanol, Ethanol, n-Propanol, iso-Propanol, n-Butanol, 2-Butanol (sec- Butanol) 2-Methyl-1-propanol (iso-Butanol), Dichlormethan, Methylacetat, Ethylacetat.

13. Verfahren nach einem der Ansprüche 1 bis 9, wobei das Azeotrop bildende organische Lösungsmittel in Schritt IVb) dabei ausgewählt ist aus der Gruppe von Toluol, o-, m- und p-Xylol sowie Ethylbenzol und deren Gemischen, bevorzugt sind o-, mund p-Xylol und deren technische Gemische, die bis zu 25 Gew.-% Ethylbenzol enthalten können.

14. Verfahren nach einem der Ansprüche 1 bis 13, wobei die Destillation in Schritt V bei einem Druck von 2 bis 6 mbar durchgeführt wird.

## Claims

1. A process for the preparation of (S)-2-amino-1-propanol comprising the following steps:
I) reaction of (S)-1-methoxy-2-propylamine with at least 2 equivalents of a 30 to 40% strength by weight hydrochloric acid, either
Ia) at temperatures greater than 80°C in an autoclave at pressures in the range from 3 to 45 bar for 1 to 12 hours and subsequent cooling to room temperature and decompression of the autoclave or
Ib) it is heated at reflux for 30 to 60 hours at atmospheric pressure,
II) then the aqueous solvent is distilled off from step Ia) or from step Ib)
III) then the reaction product from step II) is either
IIIa) admixed with an inorganic base until a pH greater than 10 has been established
or
IIIb) the product obtained from step II is reacted with a mixture of a relatively high-boiling solvent and a relatively strong base,
IV) then
either
IVa) the reaction product from step IIIa is freed from the water by distillation and the residue is admixed with a solvent and then filtered,
or
IVb) the reaction product from step IIIa is admixed with a mixture of an azeotrope-forming organic solvent and a relatively high-boiling diluent, and water and (S)-2-aminopropan-1-ol are distilled off azeotropically together with the azeotrope-forming organic solvent, then the (S)-2-aminopropan-1-ol-comprising distillation fractions are combined,
V) the filtrate obtained from step IVa) or the combined filtrates obtained from step IVb) or the mixtures obtained from step IIIb) are distilled.

2. The process according to claim 1, where a 35 to 38% strength hydrochloric acid is used in step I.

3. The process according to either of claims 1 and 2, where, at a temperature in the range from 80 to 100°C, the reaction in step Ia) lasts more than 9 hours and the pressure is between 3 and 5 bar.

4. The process according to either of claims 1 and 2, where, at a temperature in the range of more than 100°C, the reaction in step Ia) lasts less than 9 hours and the pressure is between 15 and 45 bar.

5. The process according to either of claims 1 and 2, where, at a temperature in the range from 130 to 135°C, the reaction in step Ia) lasts less than 4.5 hours and the pressure is between 19-30 bar.

6. The process according to either of claims 1 and 2, where, according to step Ib), heating is carried out at reflux for 45-60 hours.

7. The process according to either of claims 1 and 2, where, according to step Ib), after 15 hours, a further 1-2 equivalents of a 30 to 40% strength by weight hydrochloric acid are added and heating is carried out at reflux for a total of 30-50 hours.

8. The process according to any one of claims 1 to 7, where the inorganic base used in step IIIa) is selected from the group of sodium hydroxide, potassium hydroxide, lithium hydroxide, calcium hydroxide, sodium carbonate, potassium carbonate, potassium hydrogencarbonate or sodium hydrogencarbonate.

9. The process according to any one of claims 1 to 8, where the pH in step IIIa is greater than or equal to 12.

10. The process according to any one of claims 1 to 7, where the relatively high-boiling solvent used in step IIIb is selected from the group of glycols having 2 to 8 carbon atoms, such as ethylene glycol, diethylene glycol and triethylene glycol or polyalkylene glycols, or amino alcohols, such as ethanolamine, diethanolamine and triethanolamine, or mixtures of these solvents, and also mixtures thereof with water.

11. The process according to any one of claims 1 to 7 and 10, where the relatively strong base used in step IIIb is selected from the group of alkali metal hydroxides such as sodium hydroxide, potassium hydroxide, or alkaline earth metal hydroxides such as calcium hydroxide, or alkali metal alcoholates, such as sodium methanolate, sodium ethanolate, potassium ethanolate, sodium tert-butanolate and potassium tert-butanolate or diazabicyclooctane (DABCO), diazabicyclononane (DBN), diazabicycloundecane (DBU) and tri-n-octylamine.

12. The process according to any one of claims 1 to 9, where the solvent which is used in step IVa) is selected from the group of methanol, ethanol, n-propanol, isopropanol, n-butanol, 2-butanol (sec-butanol), 2-methyl-1-propanol (isobutanol), dichloromethane, methyl acetate, ethyl acetate.

13. The process according to any one of claims 1 to 9, where the azeotrope-forming organic solvent in step IVb) is selected from the group of toluene, o-, m- and p-xylene, and also ethylbenzene and mixtures thereof, preference being given to o-, m- and p-xylene and technical-grade mixtures thereof which can comprise up to 25% by weight of ethylbenzene.

14. The process according to any one of claims 1 to 13, where the distillation in step V is carried out at a pressure of from 2 to 6 mbar.

## Revendications

1. Procédé de fabrication de (S)-2-amino-1-propanol comprenant les étapes suivantes :
I) la mise en réaction de (S)-1-méthoxy-2-propylamine avec au moins 2 équivalents d'un acide chlorhydrique de 30 à 40 % en poids,
soit
Ia) à des températures supérieures à 80 °C dans un autoclave à des pressions dans la plage allant de 3 à 45 bar pendant 1 à 12 heures, avec refroidissement ultérieur à température ambiante et détente de l'autoclave,
soit
Ib) avec chauffage à reflux pendant 30 à 60 heures à pression normale,
II) puis l'élimination par distillation du solvant aqueux de l'étape Ia) ou de l'étape Ib),
III) puis
soit
IIIa) le mélange du produit de réaction de l'étape II) avec une base inorganique jusqu'à l'ajustement d'un pH supérieur à 10,
soit
IIIb) la mise en réaction du produit obtenu à l'étape II avec un mélange d'un solvant de point d'ébullition plus élevé et d'une base plus forte,
IV) puis
soit
IVa) l'élimination par distillation de l'eau du produit de réaction de l'étape IIIa et le mélange du résidu avec un solvant, puis sa filtration,
soit
IVb) le mélange du produit de réaction de l'étape IIIa avec un mélange d'un solvant organique formant un azéotrope et d'un diluant de point d'ébullition plus élevé, et l'élimination de l'eau et du (S)-2-aminopropan-1-ol ensemble par distillation azéotropique avec le solvant organique formant un azéotrope, puis la réunion des fractions de distillation contenant du (S)-2-aminopropan-1-ol,
V) la distillation du filtrat obtenu à l'étape IVa) ou des filtrats réunis obtenus à l'étape IVb) ou des mélanges obtenus à l'étape IIIb).

2. Procédé selon la revendication 1, dans lequel un acide chlorhydrique de 35 à 38 % est utilisé à l'étape I.

3. Procédé selon l'une quelconque des revendications 1 à 2, dans lequel la réaction à l'étape Ia) dure plus de 9 heures et la pression est comprise entre 3 et 5 bar à une température dans la plage allant de 80 à 100 °C.

4. Procédé selon l'une quelconque des revendications 1 à 2, dans lequel la réaction à l'étape Ia) dure moins de 9 heures et la pression est comprise entre 15 et 45 bar à une température dans la plage supérieure à 100 °C.

5. Procédé selon l'une quelconque des revendications 1 à 2, dans lequel la réaction à l'étape Ia) dure moins de 4,5 heures et la pression est comprise entre 19 et 30 bar à une température dans la plage allant de 130 à 135 °C.

6. Procédé selon l'une quelconque des revendications 1 à 2, dans lequel le chauffage à reflux selon l'étape Ib) est effectué pendant 45 à 60 heures.

7. Procédé selon l'une quelconque des revendications 1 à 2, dans lequel, selon l'étape Ib), 1 à 2 équivalents supplémentaires d'un acide chlorhydrique de 30 à 40 % en poids sont ajoutés après 15 heures, et le mélange est porté à reflux pendant au total 30 à 50 heures.

8. Procédé selon l'une quelconque des revendications 1 à 7, dans lequel la base inorganique utilisée à l'étape IIIa) est choisie dans le groupe constitué par l'hydroxyde de sodium, l'hydroxyde de potassium, l'hydroxyde de lithium, l'hydroxyde de calcium, le carbonate de sodium, le carbonate de potassium, l'hydrogénocarbonate de potassium ou l'hydrogénocarbonate de sodium.

9. Procédé selon l'une quelconque des revendications 1 à 8, dans lequel le pH à l'étape IIIa est supérieur ou égal à 12.

10. Procédé selon l'une quelconque des revendications 1 à 7, dans lequel le solvant de point d'ébullition plus élevé utilisé à l'étape IIIb est choisi dans le groupe constitué par les glycols contenant 2 à 8 atomes de carbone tels que l'éthylène glycol, le diéthylène glycol et le triéthylène glycol ou les polyalkylène glycols ou les aminoalcools tels que l'éthanolamine, la diéthanolamine et la triéthanolamine ou les mélanges de ces solvants, ainsi que leurs mélanges avec l'eau.

11. Procédé selon l'une quelconque des revendications 1 à 7 et 10, dans lequel la base plus forte utilisée à l'étape IIIb est choisie dans le groupe constitué par les hydroxydes alcalins tels que l'hydroxyde de sodium, l'hydroxyde de potassium ou les hydroxydes alcalino-terreux tels que l'hydroxyde de calcium ou les alcoolates alcalins tels que le méthanolate de sodium, l'éthanolate de sodium, l'éthanolate de potassium, le tert.-butanolate de sodium et le tert.-butanolate de potassium ou le diazabicyclooctane (DABCO), le diazabicyclononane (DBN), le diazabicycloundécane (DBU) et la tri-n-octylamine.

12. Procédé selon l'une quelconque des revendications 1 à 9, dans lequel le solvant utilisé à l'étape IVa) est choisi dans le groupe constitué par le méthanol, l'éthanol, le n-propanol, l'isopropanol, le n-butanol, le 2-butanol (sec-butanol), le 2-méthyl-1-propanol (isobutanol), le dichlorométhane, l'acétate de méthyle, l'acétate d'éthyle.

13. Procédé selon l'une quelconque des revendications 1 à 9, dans lequel le solvant organique formant un azéotrope à l'étape IVb) est choisi dans le groupe constitué par le toluène, l'o-, le m- et le p-xylène, ainsi que l'éthylbenzène et leurs mélanges, de préférence l'o-, le m- et le p-xylène et leurs mélanges techniques, qui peuvent contenir jusqu'à 25 % en poids d'éthylbenzène.

14. Procédé selon l'une quelconque des revendications 1 à 13, dans lequel la distillation à l'étape V est réalisée à une pression de 2 à 6 mbar.
